# EUROPÄISCHE PATENTSCHRIFT

(11) **EP 1 161 413 B1**
(45) Veröffentlichungstag und Bekanntmachung des Hinweises auf die Patenterteilung: **13.08.2003**
(21) Anmeldenummer: 00909259.4
(22) Anmeldetag: 28.02.2000
(51) Int. Cl.: C07C 239/20

(54) **VERFAHREN ZUR KONTINUIERLICHEN HERSTELLUNG VON METHOXYAMIN-HYDROCHLORID**
METHOD FOR THE CONTINUOUS PRODUCTION OF METHOXYAMINE HYDROCHLORIDE
PROCEDE DE PRODUCTION CONTINUE D'HYDROCHLORURE DE METHOXYAMINE

(30) Priorität: 15.03.1999 DE 19911234
(43) Veröffentlichungstag der Anmeldung: 12.12.2001
(73) Patentinhaber: BASF AKTIENGESELLSCHAFT, 67056 Ludwigshafen (DE)
(72) Erfinder: HEIMANN, Frank, D-67065 Ludwigshafen (DE); PESCHEL, Werner, D-67251 Freinsheim (DE); BARTENBACH, Bernd, D-67117 Limburgerhof (DE); HARTMANN, Horst, D-67459 Böhl-Iggelheim (DE); KEIL, Michael, D-67251 Freinsheim (DE); WAHL, Josef, D-67105 Schifferstadt (DE)
(74) Vertreter: Fitzner, Uwe, Dr.
(86) Internationale Anmeldenummer: EP0001638
(87) Internationale Veröffentlichungsnummer: WO00055121

(56) Entgegenhaltungen:
- EP-A- 0 121 701
- EP-A- 0 259 850
- EP-A- 0 440 582
- EP-A- 0 591 798
- EP-A- 0 708 082

## Beschreibung

Die vorliegende Erfindung betrifft ein Verfahren zur kontinuierlichen Herstellung von Methoxyamin-Hydrochlorid durch Spaltung von Acetonoximmethylether mit Chlorwasserstoff in Gegenwart von Wasser..

Im Stand der Technik sind sowohl diskontinuierliche Verfahren(EP-A 591 798, EP-A 708 082) als auch ein kontinuierliches Verfahren (EP-A 259 850) zur Herstellung von Methoxyamin-Hydrochlorid ausgehend von Acetonoximmethylether beschrieben. Die Vorteile eines kontinuierlichen Verfahrens liegen in der Regel in der kleineren Dimensionierung der Apparate, dem höheren Automatisierungsgrad und insbesondere der erhöhten Prozeßfähigkeit begründet. Mit kontinuierlichen Verfahren hergestellte großtechnische Produkte sind dadurch zumeist wirtschaftlicher als die mit diskontinuierlichen Verfahren gewonnenen Produkte. Weiterhin lassen sich mit kontinuierlichen Verfahren konstant hohe Produktqualitäten erreichen.

Das aus EP-A 259 850 bekannte kontinuierliche Verfahren zur Spaltung von Acetonoximmethylether zu Methoxyamin-Hydrochlorid mit Chlorwasserstoff hat jedoch den großen Nachteil, daß zur Erzielung von hohen Ausbeuten eine Reaktionskolonne mit einer Vielzahl theoretischer Trennstufen vonnöten ist. Für eine derartige Anlage ist ein hoher apparativer Aufwand sowie zum Betreiben ein großer Energieaufwand notwendig.

Aufgabe der vorliegenden Erfindung war es demnach ein kontinuierliches Verfahren zu entwickeln, das den in EP-A 259 850 beschriebenen Nachteil nicht aufweist.

Demgemäß wurde das eingangs erwähnte Verfahren gefunden, das dadurch gekennzeichnet ist, daß man die Spaltung in einer Reaktionskolonne von weniger als 20 theoretischen Trennstufen durchführt und eine Kopfabzugsmenge von mindestens 30 % der Zulaufmenge einstellt.

Das bei der Reaktion gebildete Aceton, überschüssiger Chlorwasserstoff und Wasser werden über Kopf abgezogen. Im erfindungsgemäßen Verfahren ist es wesentlich, daß über Kopf mindestens 30 % der Zulaufmenge abgezogen werden, da ansonsten vorallem in den oberen Segmenten der Reaktionskolonne die Acetonkonzentration zu hoch wäre. Eine hohe Acetonkonzentration beeinträchtigt die Lage Reaktionsgleichgewichts und führt ferner zum Absinken der Innentemperatur in diesen Segmenten. Führt man die Spaltung des Acetonoximmethylethers in einer Reaktionskolonne mit weniger als 20 theoretischen Trennstufen durch und reichert das Aceton nicht genügend ab, so erhält man das Spaltprodukt Methoxyamin-Hydrochlorid in völlig unbefriedigenden Ausbeuten.

Die erfindungsgemäße Fahrweise, daß mindestens 30 % der Zulaufmenge über Kopf abgezogen wird, führt zu einer homogenen Temperaturverteilung über die Kolonne. In der Regel beträgt die Temperaturdifferenz am Boden und am Kopf (gemessen an der vorletzten Trennstufe) weniger als 15°C und vorzugsweise weniger als 10°C.

Überraschenderweise läßt sich durch die erfindungsgemäße Fahrweise auch in einer Kolonne mit weniger als 20 theoretischen Trennstufen Methoxyamin-Hydrochlorid in exzellenten Ausbeuten erhalten.

Vorzugsweise wird im erfindungsgemäßen Verfahren das Gemisch am Kolonnenkopf mit einem fest eingestellten Rücklaufverhältnis von 2 bis 15 und vorzugsweise 3 bis 11 abgezogen. Mit der geringen Anzahl von theoretischen Trennstufen und dem günstigen Rücklaufverhältnis geht eine im Vergleich zur EP-A 259 850 deutlich kürzere mittlere Verweilzeit der Reaktionsmischung in der Kolonne einher. Überraschenderweise lassen sich im erfindungsgemäßen Verfahren trotz der kurzen mittleren Verweildauer von lediglich 20 Minuten exzellente Ausbeuten realisieren. Längere Verweilzeiten in der Kolonne sind jedoch ebenfalls möglich.

Das erfindungsgemäße Verfahren wird im folgenden näher erläutert.

Die Ausgangsverbindung Acetonoximmethylether wird nach literaturbekannten Verfahren, insbesondere nach dem in EP-A 708 082 beschriebenen Verfahren hergestellt.

Die Spaltung wird durch Zugabe von mindestens 22%iger wässriger Salzsäure erreicht. Insbesondere wird die Spaltung in Salzsäure einer Konzentration von 22 bis 38 Gew% und vorzugsweise 30 bis 38 Gew% (konzentrierte Salzsäure) durchgeführt. Die Spaltung kann jedoch auch durch Einleitung von gasförmigem Chlorwasserstoff durchgeführt werden. Im diesem Fall wird dem Reaktionsgemisch soviel Wasser zugesetzt, daß das gebildete Methoxyamin-Hydrochlorid in der Kolonne nicht auskristallisiert. In der Regel genügen hierfür 2 Moläquivalente Wasser in Bezug auf den eingesetzten Acetonoximmethylether.

Der in Form von Salzsäure oder gasförmigem Chlorwasserstoff zugeführte Chlorwasserstoff wird in Bezug auf den Acetonoximmethylether in der Regel im Überschuß, mindestens jedoch stöchiometrischen Mengen eingesetzt. Vorzugsweise wird mit einem Molverhältnis von Chlorwasserstoff zu Acetonoximmethylether von 2:1 gearbeitet.

Insbesondere bevorzugt wird eine Lösung des Acetonoximmethylethers in wässriger Salzsäure eingesetzt.

Als Reaktionskolonnen eignen sich besonders Bodenkolonnen aller Bauarten. Insbesondere sind Glockenbodenkolonnen geeignet, da sich in ihnen die Verweilzeiten auf den Böden gut einstellen lassen. Gepackte Kolonnen kommen auch in Betracht. Die Zahl der theoretischen Trennstufen (Böden) ist aus wirtschaftlichen Gründen auf 19 beschränkt. Die Mindestzahl der Trennstufen, die in der Kolonne erforderlich sind, um vollständigen Umsatz zu garantieren, hängt von dem am Kolonnenkopf eingestellten Rücklaufverhältnis ab. Da wie eingangs erwähnt vorzugsweise ein Rücklaufverhältnis von 2 bis 15 eingestellt wird, ergibt sich für die Reaktionskolonne eine Mindestanzahl theoretischer Trennstufen von 8. Wählt man hingegen ein Rücklaufverhältnis von 16 und größer, so kann Acetonoximmethylether auch in Reaktionskolonnen mit weniger als 8 Trennstufen vollständig gespalten werden.

Die Wahl des Aufgabebodens wird in den Beispielen exemplarisch dargestellt. Sie erfolgt im allgemeinen so, daß wenigstens 60 % der Böden bzw. Trennstufen über und wenigstens 20 % der Böden bzw. Trennstufen unter dem Zulauf liegen. Von dieser sinnvollen Vorgabe kann allerdings auch abgewichen werden, da durch geeignete Wahl der anderen Parameter, wie Rücklaufverhältnis, Temperatur und Kopfabzugsmenge das Verfahrensziel trotzdem erreicht werden kann.

Die Reaktionstemperatur beträgt im allgemeinen 40 bis 110° und vorzugsweise 65 bis 95°C und ergibt sich insbesondere aus der Kopfabzugsmenge und dem eingestellten Druck. Vorzugsweise wird bei einem Druck von 100 mbar bis 3 bar gearbeitet.

Die Reaktionspartner Acetonoximmethylether, Wasser und Chlorwasserstoff bzw. wässrige Salzsäure werden zweckmäßigerweise in den Mittelteil der Kolonne eingespeist. Zulaufgeschwindigkeit und Sumpfheizleistung werden im allgemeinen so gewählt, daß die mittlere Verweilzeit in der Kolonne 20 Minuten bis 2 Stunden beträgt.

Weiterhin kann die Spaltung auch in Gegenwart eines inerten organischen Lösungsmittel durchgeführt werden. Das Lösungsmittel wird vorzugsweise über Kopf zurückgewonnen und sollte daher einen Siedepunkt von maximal 150°C aufweisen. Wird mit wässriger Salzsäure gespalten, so bieten sich insbesondere Lösungsmittel wie Toluol an, die mit wässriger Salzsäure ein Azeotrop bilden.

Methoxyamin-Hydrochlorid fällt im Sumpf der Kolonne vorzugsweise als wässrige Suspension oder Lösung an, die kontinuierlich ausgetragen und beispielsweise durch Verdamfen des Verdünnungsmittels oder durch Kristallisation in reiner Form erhalten werden kann. Bei allen Isolierungsschritten zu reinem Methoxyamin-Hydrochlorid sollte aus Sicherheitsgründen eine Temperatur über 60°C nicht überschritten werden. Bevorzugt verarbeitet man die erhaltenen wässrige Lösung von Methoxyamin-Hydrochlorid weiter und verzichtet auf eine Isolierung. Die Ausbeute liegt in der Regel bei >98%.

Methoxyamin-Hydrochlorid ist ein wichtiges Zwischenprodukt zur Herstellung von Arzneimitteln und Pflanzenschutzmitteln.

### Herstellung von Methoxyamin-Hydrochlorid

### Beispiel 1

In eine Glockenbodenkolonne mit 15 theoretischen Trennstufen (entsprechend 25 praktischen Böden) und 30 mm Innendurchmesser wurden am 11. praktischen Boden kontinuierlich 400 g/h einer Lösung von 26,1 Gew% Acetonoximmethylether in 31%iger Salzsäure gefahren. Die Kolonne war mit einem Dünnschichtverdampfer und einem automatischen Rücklaufteiler ausgestattet. Das Rücklaufverhältnis war fest auf 5 eingestellt. Die Sumpfheizleistung wurde so gewählt, daß sich - bei einem Druck von 450 mbar im Kolonnenkopf - auf dem 23. praktischen Boden eine Temperatur > 84°C ergab. Im Kolonnensumpf lag die Temperatur bei 95°C. Auf diese Weise konnten im Sumpf 280 g/h (maximal 70% des Zulaufs) einer wässrig-salzsauren Lösung von 35,1 Gew% Methoxyaminhydrochlorid (entsprechend 98,2% Ausbeute) kontinuierlich abgezogen werden. Das Kopfdestillat enthielt hauptsächlich Aceton neben Wasser und gelöstem Chlorwasserstoff.

### Beispiel 2

In eine Glockenbodenkolonne mit 15 theoretischen Trennstufen (entsprechend 25 praktischen Böden) und 50 mm Innendurchmesser wurden am 11. praktischen Boden kontinuierlich 500 g/h einer Lösung von 27,5 Gew% Acetonoximmethviether in konzentrierter Salzsäure gefahren. Das Rücklaufverhältnis war fest auf 3 eingestellt. Die Sumpfheizleistung wurde so gewählt, daß sich - bei einem Druck von 450 mbar im Kolonnenkopf - auf dem 22. praktischen Boden eine Temperatur > 84°C ergab. Im Kolonnensumpf lag die Temperatur bei 98°C. Auf diese Weise konnten im Sumpf 350 g/h (maximal 70% des Zulaufs) einer wässrig-salzsauren Lösung von 37,5 Gew% Methoxyaminhydrochlorid (entsprechend 99,5% Ausbeute) kontinuierlich abgezogen werden. Das Kopfdestillat enthielt hauptsächlich Aceton neben Wasser und gelöstem Chlorwasserstoff.

### Beispiel 3

Die Spaltung wurde in einer Glockenbodenkolonne mit 9 theoretischen Trennstufen und einem Rücklaufverhältnis 11 bei ansonsten gleichen Bedingungen wie Beispiel 1 durchgeführt. Es wurde ein mit Beispiel 1 vergleichbares Ergebnis erzielt.

### Beispiel 4 (Vergleichsbeispiel)

In eine Glockenbodenkolonne mit 18 theoretischen Trennstufen (entsprechend 30 praktischen Böden) und 50 mm Innendurchmesser wurden am 16. Boden kontinuierlich 500 g/h einer Lösung von 29,3 Gew% Acetonoximmethylether in konzentrierter Salzsäure gefahren. Die Kolonne war mit einem Dünnschichtverdampfer und einem automatischen Rücklaufteiler ausgestattet. Das Rücklauf verhältnis war fest auf 4 eingestellt. Die Sumpfheizleistung wurde so gewählt, daß sich - bei einem Druck von 450 mbar im Kolonnenkopf - auf dem 28. praktischen Boden eine Temperatur < 80°C ergab. Im Kolonnensumpf lag die Temperatur dabei bei 97°C. Auf diese Weise konnten im Sumpf 380 g/h (76% des Zulaufs) einer wässrig-salzsauren Lösung von 32,6 Gew% Methoxyaminhydrochlorid (entsprechend 88,1% Ausbeute) kontinuierlich abgezogen werden. Das Kopfdestillat enthielt hauptsächlich Aceton neben Wasser, gelöstem Chlorwasserstoff. In Kopf- bzw. Sumpfaustrag wurden-nennenswerte Gehalte von nicht umgesetztem Acetonoximmethylether (3,4 bzw. 3,5 Gew%) gefunden.

## Patentansprüche

1. Verfahren zur kontinuierlichen Herstellung von Methoxyamin-Hydrochlorid durch Spaltung von Acetonoximmethylether mit Chlorwasserstoff und Wasser, **dadurch gekennzeichnet, daß** man die Spaltung in einer Reaktionskolonne von weniger als 20 theoretischen Trennstufen durchführt und eine Kopfabzugsmenge von mindestens 30 % der Zulaufmenge einstellt und Chlorwasserstoff und Wasser eine mindestens 22 gew.-%ige Salzsäure bedeuten.

2. Verfahren gemäß den Anspruch 1, **dadurch gekennzeichnet, daß** der Chlorwasserstoff und Wasser in Form von 22 bis 38 gew.%iger wäßriger Salzsäure eingesetzt werden.

3. Verfahren gemäß den Ansprüchen 1 und 2, **dadurch gekennzeichnet, daß** eine Innentemperatur von 40 bis 110°C eingestellt wird.

## Claims

1. A process for the continuous preparation of methoxyamine hydrochloride by cleavage of acetone oxime methyl ether by means of hydrogen chloride and water, wherein the cleavage is carried out in a reaction column having less than 20 theoretical plates and the amount taken off at the top is set to at least 30% of the amount of feed, and hydrogen chloride and water means a hydrochloric acid having a concentration of at least 22% by weight.

2. A process as claimed in claim 1, wherein the hydrogen chloride and water are used in the form of aqueous hydrochloric acid having a concentration of from 22 to 38% by weight.

3. A process as claimed in claim 1 or 2, wherein an internal temperature of from 40 to 110°C is set.

## Revendications

1. Procédé de préparation en continu d'hydrochlorure de méthoxyamine par décomposition d'éther méthylique d'acétonoxime par du chlorure d'hydrogène en présence d'eau, **caractérisé en ce que** l'on entreprend le clivage dans une colonne de réaction comportant moins de 20 étages de séparation théoriques et que l'on ajuste une quantité de soutirage en tête de colonne d'au moins 30% de la quantité d'alimentation, et que le chlorure d'hydrogène et l'eau représentent de l'acide chlorhydrique à au moins 22% en poids.

2. Procédé selon la revendication 1, **caractérisé en ce que** le chlorure d'hydrogène et l'eau sont mis en oeuvre sous la forme d'acide chlorhydrique aqueux à 22 à 38% en poids.

3. Procédé selon les revendications 1 et 2, **caractérisé en ce que** l'on ajuste une température interne de 40 à 110°C.
